# EUROPEAN PATENT APPLICATION

(11) **EP 4 446 335 A1**
(43) Date of publication of application: **16.10.2024**
(21) Application number: 23855750.8
(22) Date of filing: 27.10.2023
(51) Int. Cl.: C07K 7/08, C07K 7/06, A61P 25/04, A61K 38/00

(54) **NOVEL PEPTIDE AND USE THEREOF**

(30) Priority: 28.02.2023 KR 20230026404
(71) Applicant: Rudacure Corporation, Incheon 21988 (KR)
(72) Inventor: KIM, Yong Ho, Incheon 21988 (KR); KIM, Seung Hoon, Incheon 21988 (KR); KIM, Min Jung, Incheon 21988 (KR); PARK, Juil, Incheon 21988 (KR); JEON, Hawon, Incheon 21988 (KR); JANG, Yewon, Incheon 21988 (KR)
(74) Representative: Louis Pöhlau Lohrentz
(86) International application number: PCT/KR2023/016946
(87) International publication number: WO 2024/181636

(57) **Abstract**

A novel peptide is disclosed.

## Description

### TECHNICAL FIELD

The present invention relates to a novel peptide and a novel pharmaceutical composition including the same. More specifically, the present invention relates to a novel peptide and a pharmaceutical composition for treating TRPV1 activity-mediated diseases (e.g., pain, etc.) including the same, or a method of treating TRPV1 activity-mediated diseases using the peptide.

### BACKGROUND ART

Transient receptor potential vanilloid type 1 (TRPV1) is a non-selective Ca2+-permeable cation channel that may be activated by an exogenous factor (e.g. capsaicin, resiniferatoxin, etc.), an endogenous factor (e.g. arachidonic acid metabolite, anandamide, etc.) or a physical and chemical stimulus such as heat or pH, and is a key protein involved in the development of peripheral and central sensitization.

TRPV1 is proposed as a major target for the treatment of chronic pain, and research on TRPV1 agonists and antagonists is actively carried out.

TRPV1 is mainly present in sensory nerves, but it is also present in other tissues such as the brain, bronchi, kidneys, bladder, epithelial cells, and epidermal keratinocytes.

Recently, TRPV1-mediated diseases are being actively studied.

It is known that GDF11 protein is involved in the TRPV1 channel and can be used to treat TRPV1 activity-mediated diseases. However, since the GDF11 protein consists of a 109-amino acid sequence, there are still difficulties in producing the GDF11 protein industrially and using it as a medicine.

Accordingly, there is a need to develop a peptide capable of treating TRPV1-mediated diseases as a peptide with a relatively short length so that it can be used for medicinal purposes in replacement of GDF 11.

### DETAILED DESCRIPTION OF THE INVENTION

### TECHNICAL PROBLEM

The present inventors studied to develop a peptide capable of treating TRPV1-mediated diseases as a peptide with a relatively short length so that it can be used for medicinal purposes instead of GDF11, and as a result, the inventors found that even a peptide with a short length has a therapeutic effect on TRPV1-mediated diseases.

In particular, by treating and alleviating pain which is a representative TRPV1-mediated disease, it may be effectively used for use in treatment of pain that persists despite disappearing of the stimuli and clear healing of the damaged region, or pain that occurs despite the absence of any stimulus, damage, or disease.

### TECHNICAL SOLUTION

The present invention is intended to solve the above problems, and relates to a peptide including any one sequence of SEQ ID NOS: 1 to 49, and preferably, it may consist of 25 amino acids or less.

In addition, the present invention relates to a peptide including any one sequence represented by Formula 1 below, and preferably, it may consist of 25 amino acids or less.

[Formula 1] X1-X2-X3-X4-Y-M-X7-X8-Q-X10-W

wherein
X1 is A, S or Y,
X2 is G, K, S or L,
X3 is Q, M, R, I, T, L or S,
X4 is A, C, W, M, P or G,
X7 is F, W or Y,
X8 is M, F, Y, S, G, H or P, and
X10 is any amino acid.

The peptide is preferable wherein, X1 is Y, X2 is G, X3 is Q, X4 is C, X7 is W or Y, and X8 is a hydrophobic amino acid in Formula 1. Here, the hydrophobic amino acid may be M, F or Y.

In addition, in Formula 1, the X10 may be any amino acid, but it may be preferably K, Y, W, G, E, H, S, C, A or M.

In addition, the present invention relates to oligopeptides comprisiing the peptides described above.

The peptide according to the present invention has an effect of treatment for TRPV1-mediated disease. Therefore, the present invention relates to a pharmaceutical composition for treating TRPV1-mediated diseases including the above peptide.

The TRPV1 activity-mediated diseases may be pain, hypertension, stroke, myocardial ischaemia, urinary incontinence, urinary bladder hypersensitivity, irritable bowel syndrome, fecal urgency, stomach-duodenal ulcer, gastro-esophageal reflux disease (GERD), Crohn's disease, hemorrhoids, asthma, chronic obstructive pulmonary disease, pruritus, psoriasis, tinnitus, cough, hypertrichosis, alopecia, or the like.

In addition, the pain may be nociceptive pain, psychogenic pain, inflammatory pain, or pathological pain. The pathological pain may be neuropathic pain, cancer pain, chemotherapy-induced pain, postoperative pain, trigeminal neuralgia pain, idiopathic pain, diabetic neuropathic pain, or migraine.

Therefore, the present invention relates to a pharmaceutical composition for treating TRPV1 activity-mediated diseases as described above. A pharmaceutical composition according to the present invention may be administered into the body in an oral dosage form or a parenteral dosage form (e.g., injection dosage form).

In addition, the present invention may be a polynucleotide encoding the peptide or an expression vector comprising such a polynucleotide.

Therefore, the present invention relates to a pharmaceutical composition for treating TRPV1 activity-mediated diseases comprising the above peptide, a polynucleotide encoding the same, or an expression vector comprising such a polynucleotide as an active ingredient.

The one-letter alphabets used to represent amino acids in the present specification are used to represent specific amino acids as shown in Table 1 below, respectively:

**[Table 1] One-letter alphabets representing specific amino acid**

| Amino acid | Three-letter abbreviation | **One-letter abbreviation** |
|---|---|---|
| Alanine | Ala | A |
| Arginine | Arg | R |
| Asparagine | Asn | N |
| Aspartic Acid | Asp | D |
| Cysteine | Cys | C |
| Glutamine | Gln | Q |
| Glutamic Acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Asparagine or aspartic acid | Asx | B |
| Glutamine or glutamic acid | Glx | Z |

In the present specification, "growth differentiation factor 11 (GDF11)" may be represented by SEQ ID NO: 50, and is also referred to as bone morphogenetic protein 11 (BMP-11). It is a protein expressed by the GDF11 gene that is present on human chromosome No. 12.

### ADVANTAGEOUS EFFECTS

Since the peptide according to the present invention has a relatively short length so that it can be used for medicinal purposes in replacement of GDF11, and it can effectively inhibit TRPV1 activity, it can be effectively used as a pharmaceutical composition for treating various TRPV1 activity-mediated diseases.

### DESCRIPTION OF THE DRAWINGS

FIG. 1 shows the result of testing the effect of inhibiting calcium influx into a TRPV1 channel by peptides of SEQ ID NO: 5 and SEQ ID NO: 6 according to the present invention.
FIG. 2 shows the results of testing the effect of inhibiting calcium influx into a TRPV1 channel depending on the concentration of peptides of SEQ ID NO: 5 and SEQ ID NO: 6 according to the present invention.
FIG. 3 shows the results of testing the effect of inhibiting calcium influx into a TRPV1 channel by peptides of SEQ ID NO: 9, SEQ ID NO: 11, and SEQ ID NO: 12 according to the present invention.
FIG. 4 shows the results of testing the efficacy of a peptide according to the present invention in alleviating pain caused by formalin.
FIG. 5 shows the results of testing the efficacy of a peptide according to the present invention in alleviating pain caused by capsaicin.
FIG. 6 shows the results of testing the pain suppressing efficacy of a peptide according to the present invention in a CCI pain model.
FIG. 7 shows the results of testing the pain suppressing efficacy of a peptide according to the present invention in an SNL pain model.
FIG. 8 shows the results of testing the pain suppressing efficacy of a peptide according to the present invention in a CIPN pain model.

### MODE FOR INVENTION

Hereinafter, the present invention will be described in detail based on examples. However, the following examples are only for illustrating the present invention, and the spirit or scope of the present invention is not limited thereto.

### Example A. Design of peptides

### 1. Design of three types of peptides

TRPV1 plays an important role in acute and/or chronic pain diseases. Therefore, finding substances that antagonize TRPV1 may be a strategy for pain suppression.

The GDF11 protein of SEQ ID NO: 50 is known to be a protein that has an antagonistic effect on TRPV1.

When a peptide sequence that maintains a pain suppressing effect while reducing the length of the amino acid sequence of the GDF 11 protein is found, it can be effectively used as a medicine. Generally, when the length of a peptide sequence is short, chemical synthesis and mass production are possible, and it is advantageous in terms of production cost, and it is also expected to have high *in vivo* stability and low toxicity.

As a result of protein-protein docking simulation analysis of TRPV1 protein and GDF11 protein, it was predicted that the Gin (Q) 91 residue of GDF11 protein binds to the Tyr 513, Thr 552, and Asn 553 residues, which are the capsaicin binding sites of TRPV1.

Therefore, the present inventors designed three types of peptides, which are 11mer, 15mer, and 19mer peptides with the Gin 91 residue of GDF 11 at the center, as shown in Table 2 below:

**[Table 2]**

| SEQ ID NO. | Amino acid sequence | Description of amino acid sequence | Molecular weight (Da) |
|---|---|---|---|
| 51 | LYFNDKQQII Y | 85 to 95 residues of GDF11, 11aa | 1444.5 |
| 52 | NMLYFNDKQQ IIYGK | 83 to 97 residues of GDF11, 15aa | 1874.4 |
| 53 | PINMLYFNDK QQIIYGKIP | 81 to 99 residues of GDF11, 19aa | 2295.1 |

### 2. Modification of three types of peptides

As a result of testing the TRPV1 activity inhibiting effect of peptides obtained by modifying each of the above three types of peptides by substituting, deleting, inserting or adding one or several amino acids in the amino acid sequence, the two types of peptides shown in Table 3 below exhibited the best TRPV1 inhibition rate (test method for the effect described later):

**[Table 3]**

| SEQ ID NO. | Amino acid sequence | Length |
|---|---|---|
| 5 | YKANYCSGQC Y | 11aa |
| 6 | SGQCYMFMQK Y | 11aa |

**[Table 4]**

| SEQ ID NO. | Amino acid sequence | Note |
|---|---|---|
| 9 | AGQCYMFMQKY | Peptide sequence of SEQ ID NO: 6 partially substituted, 11aa |
| 10 | SGQAYMFMQKY | Peptide sequence of SEQ ID NO: 6 partially substituted, 11aa |
| 11 | YGQCYMWMQKW | Peptide sequence of SEQ ID NO: 6 partially substituted, 11aa |
| 12 | YGQCYMYMQKW | Peptide sequence of SEQ ID NO: 6 partially substituted, 11aa |
| 43 | YKAAYCSGQCY | Peptide sequence of SEQ ID NO: 6 partially substituted, 11aa |
| 4 4 | SGACYMFMQKY | Peptide sequence of SEQ ID NO: 6 partially substituted, 11aa |
| 45 | SGQCYAFMQKY | Peptide sequence of SEQ ID NO: 6 partially substituted, 11aa |
| 46 | SGQCYMFMQAY | Peptide sequence of SEQ ID NO: 6 partially substituted, 11aa |
| 47 | SGQCYMWMQKY | Peptide sequence of SEQ ID NO: 6 partially substituted, 11aa |
| 48 | SGQCYMWMQKW | Peptide sequence of SEQ ID NO: 6 partially substituted, 11aa |
| 49 | SGQCYMYMQKY | Peptide sequence of SEQ ID NO: 6 partially substituted, 11aa |

### 3. Design of additional peptides

As a result of selecting further peptides, including the above peptides, and testing for TRPV1 inhibitory activity as in Examples below, 49 peptides in Tables 5 and 6 below were screened as having TRPV1 activity:

**[Table 5]**

| SEQ ID NO. | Amino acid sequence |
|---|---|
| 1 | MLYFNDKQQIIY |
| 2 | PINMLYFNDKQQIIYGKID |
| 3 | GINMLYFNDKQQIIYGKIN |
| 4 | YCSGQCYMFMQKY |
| 5 | YKANYCSGQCY |
| 6 | SGQCYMFMQKY |
| 7 | YKANYCSGQCEY |
| 8 | SGQCEYMFMQKY |
| 9 | AGQCYMFMQKY |
| 10 | SGQAYMFMQKY |
| 11 | YGQCYMWMQKW |
| 12 | YGQCYMYMQKW |
| 13 | YGQCYMWMQYW |
| 14 | YGQCYMWMQWW |
| 15 | YGQCYMWMQGW |
| 16 | YGQCYMWMQKY |
| 17 | YGQCYMWMQEW |
| 18 | YGMCYMWMQKW |
| 19 | YGRCYMWMQKW |
| 20 | YGQCYMWFQKW |
| 21 | YGICYMWMQKW |
| 22 | YGQCYMWMQHW |
| 23 | YGQCYMWYQKW |
| 24 | YGQCYMWSQKW |
| 25 | YGGCYMWMQKW |
| 26 | YKQCYMWMQKW |
| 27 | YGTCYMWMQKW |
| 28 | YGQCYMWGQKW |
| 29 | YGQCYMWMQSW |
| 30 | YGQCYMWMQCW |

**[Table 6]**

| SEQ ID NO. | Amino acid sequence |
|---|---|
| 31 | YGQCYMWMQAW |
| 32 | YGQWYMWMQKW |
| 33 | YGQMYMWMQKW |
| 34 | YGLCYMWMQKW |
| 35 | YGQCYMWHQKW |
| 36 | YSQCYMWMQKW |
| 37 | YGQCYMWMQMW |
| 38 | YGQPYMWMQKW |
| 39 | YGQCYMWPQKW |
| 40 | YGQGYMWMQKW |
| 41 | YGSCYMWMQKW |
| 42 | YLQCYMWMQKW |
| 43 | YKAAYCSGQCY |
| 44 | SGACYMFMQKY |
| 45 | SGQCYAFMQKY |
| 46 | SGQCYMFMQAY |
| 47 | SGQCYMWMQKY |
| 48 | SGQCYMWMQKW |
| 49 | SGQCYMYMQKY |

The amino acid sequence according to the present invention may be configured to be protected from protease enzymes by capping an N-terminus with lipoic acid and/or capping a C-terminus with p-nitroanilide. For example, it may be synthesized by capping an N-terminus of the amino acid sequence of SEQ ID NO: 2 with lipoic acid and capping a C-terminus with p-nitroanilide.

In addition, the amino acid sequence according to the present invention may be formed in a cyclic form by connecting an N-terminus and a C-terminus with a peptide bond. For example, an N-terminus and a C-terminus of the above amino acid sequence of SEQ ID NO: 8 may be linked to each other with a peptide bond to be synthesized as a circular form.

### Example B. Evaluation of TRPV1 inhibitory effect of peptides

The TRPV1 inhibitory effect of various short-length peptides, including the 49 peptides in Tables 5 and 6, was evaluated by the following method.

### 1. Cell line culture

All reagents used in cell culture were sterilized products. The culture medium of the cell line is shown in Table 7 below, and the culture was performed in a cell incubator with under the culture conditions of 50% CO₂ and 37 °C. In addition, when cryopreserved cells (stock) were thawed and used, they were subcultured two to three times before use in the test.

**[Table 7]**

| Cell line | Culture medium composition | Culture conditions |
|---|---|---|
| TRPV1 CHO-K1 | DMEM media + 10% FBS + 1 % P/S + 400 mg/ml G418 | Incubation (5% CO₂, 37°C) |

After removing the cell culture medium by suction, 10 ml DPBS was added to wash the cells, and then the DPBS was removed by suction. 2 ml 0.05% trypsin-EDTA was added, and then the cells were cultured in a cell incubator at 37 °C for three to five minutes. Through a microscope, detachment of the cells from the bottom surface of the culture dish was confirmed, and 8 ml of the cell culture medium including 10% FBS was added to inactivate the trypsin-EDTA. Cells in the culture dish were collected, pipetted, and transferred to a 15 ml tube.

Centrifugation was performed at 800 rpm for five minutes, and all the supernatant except the cell pellet was removed. Then, the cell culture medium was added, and the pellet was carefully loosened.

After measuring the number of cells with a hemocytometer, the cells were dispensed at 1×10³ cells/10 µl/coverslip at the center of a 12 mm cover slip coated with poly-D-lysine.

A 35 π petri dish containing the cover slip was stored in a cell incubator, and when the cells were attached to the cover slip after one to two hours, 2 ml of the cell culture medium was added and stored in the cell incubator again.

### 2. Preparation of reagents and test materials

### a. Preparation of extracellular solution

The sample was added to 200 ml distilled water (DW) and mixed according to the composition in Table 8 below.

**[Table 8]**

| Extracellular solution (based on 250 ml) | | | | | |
|---|---|---|---|---|---|
| | Concentration (mM) | Amount | | Concentration (mM) | Amount |
| NaCl | 140 | 2.04 g | CaCl₂ (1 M) | 2 | 0.5 ml |
| HEPES | 10 | 0.59 g | MgCl₂ (1 M) | 1 | 0.25 ml |
| D-Glucose | 10 | 0.45 g | KCl (1 M) | 5 | 1.25 ml |

The pH was adjusted to a range of 7.3 to 7.4 using 5 N NaOH, and then distilled water (DW) was added to adjust the final volume to 250 ml.

### b. Preparation of capsaicin

Capsaicin was dissolved in 100% ethanol to prepare a 10 mM stock. Capsaicin, a TRPV1 activating substance, was diluted in the extracellular solution to a concentration of 0.1-1 mM before using in the experiment.

### c. Preparation of test materials

The test material (peptide according to the present invention) was dissolved in a solvent and then diluted to an appropriate concentration in the extracellular solution before using in the experiment.

### d. Preparation of Fura-2AM

2 mM Fura-2AM was prepared by adding 24.95 ml of DMSO to 50 mg of Fura 2AM powder, which is used as a membrane-permeable fluorescent marker. After the experiment, the remaining Fura-2AM was stored at -20 °C for reuse and covered with silver foil to prevent exposure to light.

### 3. Fura-2AM loading

2 mM Fura-2AM was diluted 1/1000 in a cell culture medium without FBS and antibiotics to prepare 1 ml Fura-2AM (based on a 35 π Petri dish) to a final concentration of 2 nM. After removing the cell culture medium in the 35 π Petri dish, the cells were washed once with 2 ml DPBS. 1 ml of the Fura-2AM solution prepared above was transferred to a 35 π Petri dish, and the cells were cultured in a cell incubator for 40 min.

### 4. Calcium image analysis

To operate calcium imaging equipment, an IRIS-9 camera was turned on and then a computer was powered on. To operate a perfusion system, a suction device was powered on, 20 to 30 ml of distilled water was added to each perfusion syringe, and suction was performed while allowing the distilled water to flow into the chamber (syringe wash).

The extracellular solution and test materials prepared in advance were placed in the syringes washed with distilled water, and smooth flow of the solutions from each syringe into the chamber was confirmed. At this time, clips were used to block all the lines except one syringe line to be checked. In other words, the flow of the solution through only a single syringe line without the clip was checked (When the solution flows through two or more lines, the solutions could have been mixed.).

Once it was confirmed that the solution was flowing well in each syringe, the test was prepared while allowing only the extracellular solution to flow until the cover slip with the attached cells was placed in the chamber.

The cells to be used in the test were placed at the center of the microscope lens and observed at a 40X magnification to set the cell region while performing calcium imaging with the filter No. 1 (bright filter). In that state, the filter was changed to the filter No. 4 (Fura-2AM wavelength filter), and the fluorescence light source (CoolLED) was turned on to observe the cells loaded with Fura-2AM. The filter No. 4 was retained to conduct the experiment further.

By running the calcium imaging driving software (MetaFluor), the F340 nm/F380 nm ratio was measured at 1-second intervals, and the test materials were treated according to the time schedule shown in the figure below to measure the level of Ca²⁺ influx according to the ratio change.

[Test material treatment time schedule]

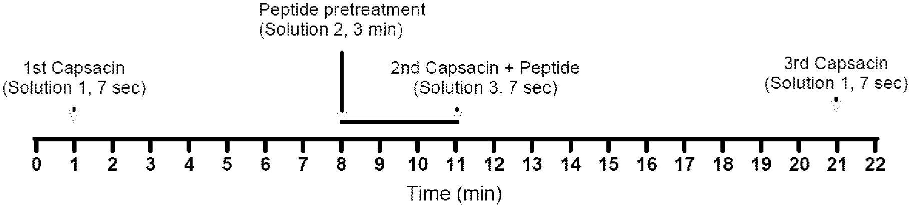
Peptide pretreatment
(Solution 2, 3 min)
1st Capsacin
(Solution 1, 7 sec)
2nd Capsacin + Peptide
(Solution 3, 7 sec)
3rd Capsacin
(Solution 1, 7 sec)
0 1 2 3 4 5 6 7 8 9 10 11 12 13 14 15 16 17 18 19 20 21 22
Time (min)

### 5. Experimental results

### a. Screening of peptides modified from 3 types of peptides (SEQ ID NOS: 51, 52, 53)

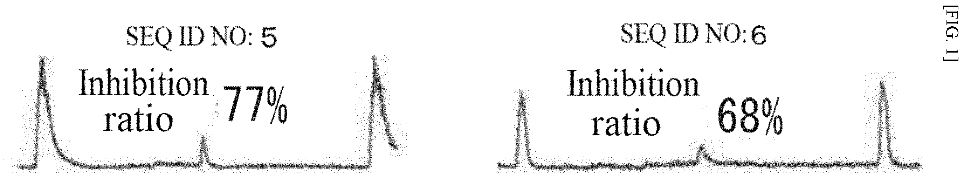

As a result of the experiment, it can be seen that when the peptide of SEQ ID NO: 5 or 6 was injected, the influx of calcium was significantly reduced (see FIG. 1: In FIG. 1, the y-axis of each graph is ΔF/F, and the x-axis is time (minutes).).

### b. Confirmation of hTRPV1 inhibitory IC50 of peptides of SEQ ID NOS: 5 and 6

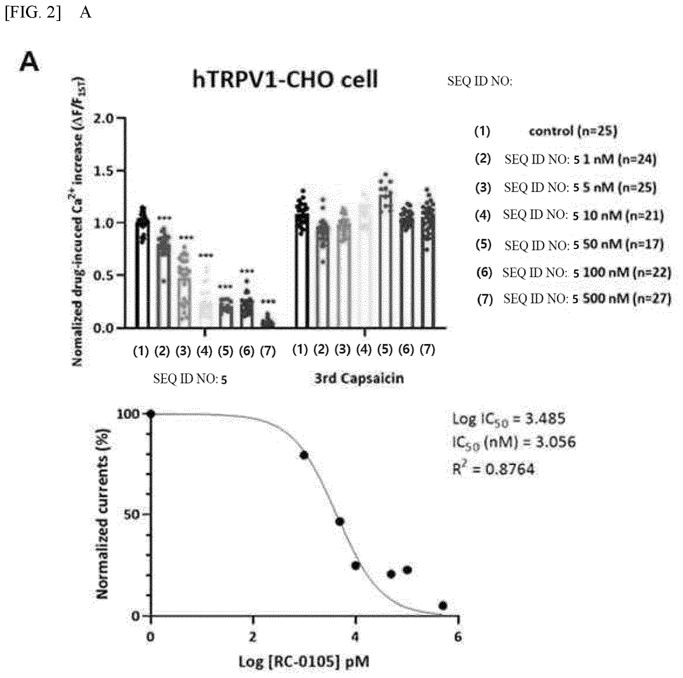

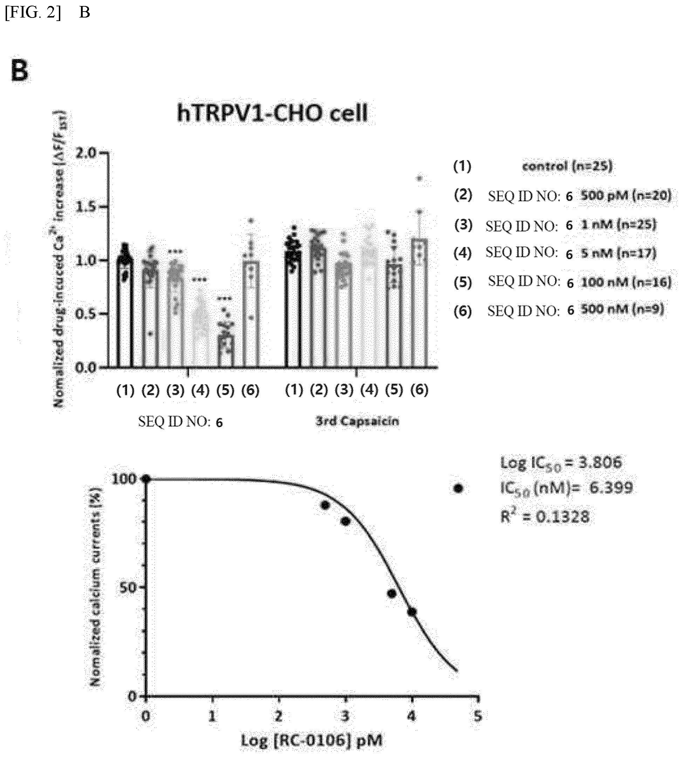

The peptides of SEQ ID NOS: 5 and 6, which exhibited high hTRPV1 inhibition efficiency, were treated at different concentrations to confirm the inhibition of hTRPV1 activity. After pre-treating the cultured cells with the peptides, the ratio value (ΔF/F) induced by capsaicin and the ratio value (ΔF/F) induced by the third capsaicin treatment were normalized based on the ratio value (ΔF/F) induced by the first capsaicin treatment. The experimental results are shown in FIG. 2.

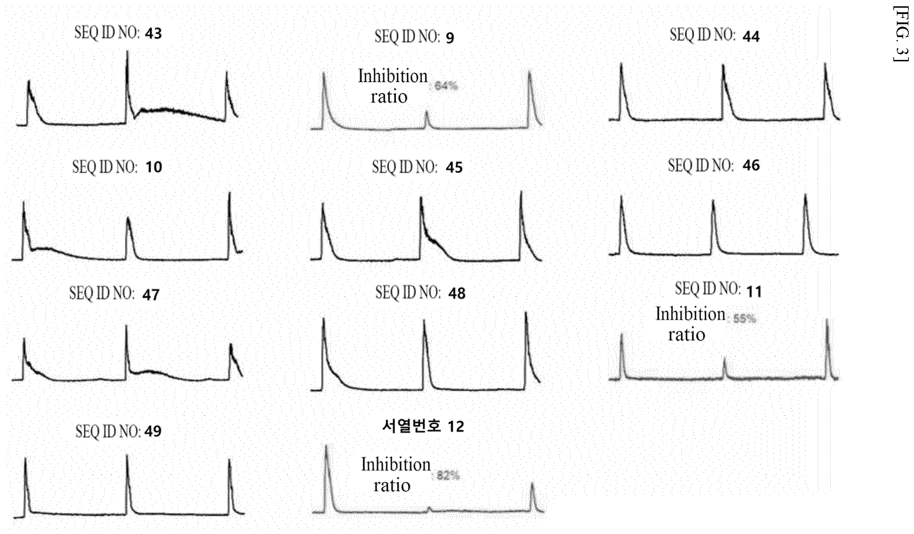

When the cells were treated with the peptide of SEQ ID NO: 5 at 1 nM, the activity was inhibited by about 20% compared to the control group, and the activity was inhibited by 53% at 5 nM, 75% at 10 nM, 79% at 50 nM, 77% at 100 nM, and 95% at 500 nM. It was confirmed that the IC50 was 3.056 nM. The experimental results are shown in FIG. 3.

When the cells were treated with the peptide of SEQ ID NO: 6 at 0.5 nM, the activity was inhibited by about 9% compared to the control group, the activity was inhibited by 16% at 1 nM, 51% at 5 nM, and 70% at 100 nM, and it was confirmed that the IC50 was 6.399 nM.

The statistical significance was analyzed by using two-way ANOVA, and it was found that *; P<005, **; p<001, and ***; p<0001 compared to the control group.

### c. Screening of peptides modified by substituting some sequences from peptides of SEQ ID NOs: 5 and 6

As a result of experiments with the amino acids of SEQ ID NO: 5 or 6 and the peptides modified by substituting one or several amino acids in each amino acid sequence of these amino acids, the peptides of SEQ ID NOS: 1 to 49 were evaluated to have TRPV1 inhibitory activity, and in particular, it was found that the peptides of SEQ ID NOS: 5, 6, 9, 11, and 12 have a high inhibition rate of TRPV1.

### Example C. Confirmation of pain alleviating effect by formalin test

An *in vivo* test was performed with the peptides of SEQ ID NOS: 11 and 12.

### 1. Test system information

**[Table 9]**

| | Description |
|---|---|
| Species | Mouse |
| Line | I C R mouse |
| Sex | male |
| Number of animals introduced | 3 2 |
| Supplier | YoungBio |
| Age | Five weeks |
| Reason for selecting the test system | An inbreeding system commonly used in non-clinical tests |

### 2. Preparation and administration of control materials

a. Preparation of negative control material: A complete saline solution product (0.9% NaCl) was used.
b. Method of administering the negative control material: The negative control material was administered intraperitoneally.
c. Number of times of administering the negative control material: The negative control material was administered once, the same as the test materials.
d. Preparation of positive control material: Pregabalin was homogeneously dissolved in a saline solution at a concentration of 3 mg/mL.
e. Method of administering the positive control material: The positive control material was administered intraperitoneally.
f. Number of times of administering the positive control material: The positive control material was administered once, the same as the test materials.

### 3. Preparation and administration of test materials

a. Preparation of test materials: The peptide of SEQ ID NO: 11 was homogeneously dissolved in a saline solution at a concentration of 0.8 mg/mL, and the peptide of SEQ ID NO: 12 was prepared in a saline solution at a concentration of 0.4 mg/mL.
b. Determination of dose of the test materials: The dose was determined considering that the typical dose of intraperitoneal administration for mice is 200 µL.
c. Administration route of the test materials: The test materials were administered intraperitoneally because it is a general route which allows for easy systematic administration and absorption.
d. Method of administering the test materials: The test materials were administered intraperitoneally using a 1 mL syringe.
e. Number of times of administering the test materials: The test materials were administered once.

### 4. Configuration of test groups

The test groups were configured as described below.

**[Table 10]**

| Group | Configuration | Administered material | Amount of administration | Dose | Administration concentration | route | n= |
|---|---|---|---|---|---|---|---|
| G1 | Negative control | Saline | - | | - | | 8 |
| G2 | Test group | SEQ ID NO: 11 | 8 mg/kg | 200 µℓ | 0.8 mg/mℓ | I.P. | 8 |
| G3 | | SEQ ID NO: 12 | 4 mg/kg | | 0.4 mg/mℓ | | 8 |
| G4 | Positive control | Pregabalin | 30 mg/kg | | 3 mg/mℓ | | 8 |

### 5. Test method

a. Saline solution, pregabalin, peptide of SEQ ID NO: 11, and peptide of SEQ ID NO: 12, the administered materials corresponding to each group, were administered in advance about one hour before formalin administration.
b. One hour after the test material administration to each group, 20 µL of 2% formalin was injected subcutaneously into the sole of the right foot.
c. After administering 2% formalin to each subject, the time at which pain behavioral responses (licking, biting, etc.) appeared was measured in the intervals of 0 to 5 minutes and 20 to 40 minutes.
d. Grooming in regions other than the region where formalin was administered was not counted.
e. Statistics were analyzed using T-test and expressed as Mean ± SEM (*=p<0.01, **=p<0.01).

### 6. Test results

In animal experiments, when formalin is injected subcutaneously, it causes continuous excitement in the dorsal horn neurons, resulting in hypersensitive behavioral responses to external stimuli. This phenomenon is referred to as central facilitation, and this symptom is referred to as hyperalgesia.

The formalin test is a method of evaluating the degree of pain by subcutaneously injecting formalin into the dorsum of the hind paw of a mouse and then observing the behavior that appears thereafter. The degree of pain is evaluated by the number of times or duration of flinching responses.

The pattern of the pain response is a typical biphasic response in which a temporary and severe pain response appears for several minutes immediately after formalin subcutaneous injection, and then the pain gradually decreases, followed by persistent and strong pain response resuming 10 minutes later. The 1st phase response is caused by direct activation of thin nociceptive afferent nerve fibers (A and C fibers) distributed in the injured tissues, while the 2^{nd} phase response is an inflammatory response, referring to the pain caused by the peripheral and central sensitization resulting from repeated stimulation of C-fibers by inflammation.

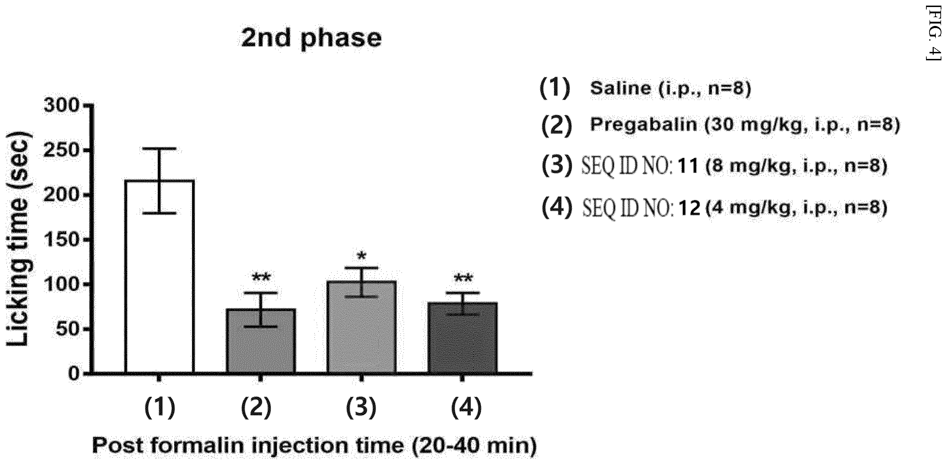

The experimental results showed that the groups administered with 8 mg/kg of the peptide of SEQ ID NO: 11 and 4 mg/kg of the peptide of SEQ ID NO: 12 exhibited a significant decrease of the pain behavioral response in the 2nd phase compared to the saline solution administration group. (See FIG. 4: Mean ± SEM, *=p<0.01, **=p<0.01).

From the above results, it could be confirmed that the administration of the peptide of SEQ ID NO: 11 or SEQ ID NO: 12 appears to inhibit the pain caused by the peripheral and central sensitization, and has a similar effect to pregabalin, which is often used as a chronic pain therapeutic.

### Example D. Confirmation of pain alleviating effect by capsaicin test

An in vivo test was performed with the peptides of SEQ ID NOS: 5, 6, 9, and 11.

### 1. Test system information

**[Table 11]**

| | Description |
|---|---|
| Species | Mouse |
| Line | C 5 7 B L / 6 N |
| Sex | male |
| Number of animals introduced | 4 4 |
| Supplier | Daehan Bio Link |
| Age | Six weeks |
| Reason for selecting the test system | An inbreeding animal commonly used in non-clinical tests |

### 2. Preparation and administration of control materials

a. Preparation of negative control material: A complete saline solution product (0.9% NaCl) was used.
b. Method of administering the negative control material: The negative control material was administered intraperitoneally.
c. Number of times of administering the negative control material: The negative control material was administered once, the same as the test materials.
d. Preparation of positive control material: BCTC was homogeneously dissolved in a saline solution at a concentration of 3 mg/mL.
e. Method of administering the positive control material: The positive control material was administered intraperitoneally.
f. Number of times of administering the positive control material: The positive control material was administered once, the same as the test materials.

### 3. Preparation and administration of test materials

a. Preparation of test materials: The peptides of SEQ ID NOS: 5, 6, 9, and 11 were dissolved in a saline solution to prepare at a concentration of 0.05 mg/mL.
b. Determination of dose of the test materials: The dose was determined considering that the typical dose of intraperitoneal administration for mice is 200 µL.
c. Administration route of the test materials: The test materials were administered intraperitoneally because it is a general route which allows for easy systematic administration and absorption.
d. Method of administering the test materials: The test materials were administered intraperitoneally using a 1 mL syringe.
e. Number of times of administering the test materials: The test materials were administered once.

### 4. Configuration of test groups

The test groups were configured as described below.

**[Table 12]**

| Configuration | Administered material | Amount of administration | Administration concentration | Dose | route | n= |
|---|---|---|---|---|---|---|
| Negative control | Saline | - | - | | | 8 |
| | SEQ ID NO: 5 | 0.5 mg/kg | 0.05 mg/mL | | | 7 |
| Test group | SEQ ID NO: 6 | 0.5 mg/kg | 0.05 mg/mL | 200µℓ | I.P | 7 |
| | SEQ ID NO: 9 | 0.5 mg/kg | 0.05 mg/mL | | | 7 |
| | SEQ ID NO: 11 | 0.5 mg/kg | 0.05 mg/mL | | | 7 |
| Positive control | BCTC | 30 mg/kg | 3 mg/mL | | | 8 |

### 5. Test method

a. Saline solution, BCTC, and peptides of SEQ ID NOS: 5, 6, 9, and 11, the administered materials corresponding to each group, were administered in advance about one hour before capsaicin administration.
b. One hour after the test material administration to each group, 20 µL of 0.01% capsaicin was injected subcutaneously into the sole of the right foot.
c. After administering 2% formalin to each subject, the time at which pain behavioral responses (licking, biting, etc.) appeared was measured in the interval of 0 to 5 minutes.
d. Grooming in regions other than the region where capsaicin was administered was not counted.
e. Statistics were analyzed using T-test and expressed as Mean ± SEM (*=p<0.05, **=p<0.01, **=p<0.001).

### 6. Test results

**Injecting** a large amount of capsaicin into laboratory animals immediately after their birth causes loss of pain nerves expressing TRPV1 channels, resulting in permanent desensitization to painful stimuli. Therefore, the capsaicin administration method has long been effectively used in studying the function of pain nerves.

In a previous study, the pain inhibiting effect was confirmed through a formalin test. To confirm whether such pain inhibiting phenomenon is through TRPV1, the pain response pattern was investigated after subcutaneous injection of capsaicin, which is one of the TRPV1 activators.

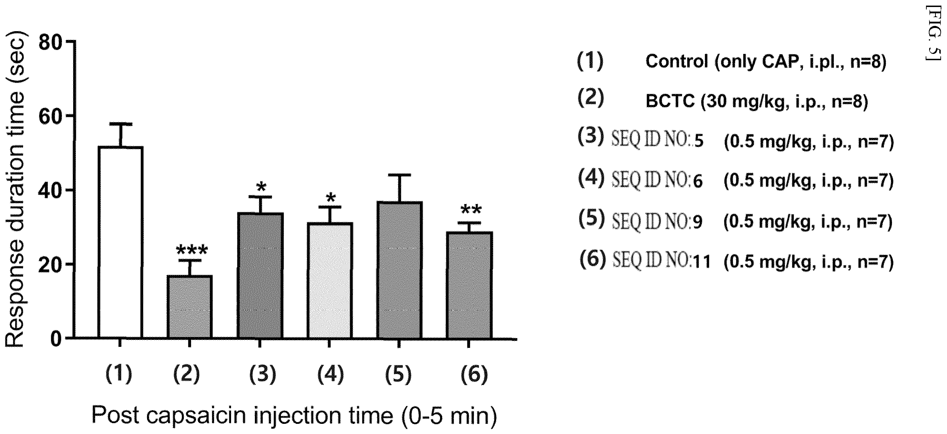

The experimental results showed that the peptides of SEQ ID NOS: 5, 6, and 11 exhibited a significant decrease of the pain behavioral response (See FIG. 5: Mean ± SEM, *=p<0.01, **=p<0.01).

From the above results, it was determined that the administration of the peptides of SEQ ID NOS: 5, 6, 9, and 11 inhibited the pain caused by the peripheral and central sensitization, and it could be confirmed that the pain inhibition by capsaicin is related to TRPV1.

### Example E. Confirmation of pain alleviating effect in CCI pain model

### 1. Test system information

**[Table 13]**

| | Description |
|---|---|
| Species | Mouse |
| Line | C 5 7 B L / 6 N |
| Sex | male |
| Number of animals introduced | 2 8 |
| Supplier | Daehan Bio Link |
| Age | Six weeks |
| Reason for selecting the test system | An inbreeding animal commonly used in non-clinical tests |

### 2. Preparation and administration of control materials

a. Preparation of negative control material: A complete saline solution product (0.9% NaCl) was used.
b. Method of administering the negative control material: The negative control material was administered intraperitoneally.
c. Number of times of administering the negative control material: The negative control material was administered once, the same as the test materials.
d. Preparation of positive control material: A complete morphine product (1 mg/mL) was used.
e. Method of administering the positive control material: The positive control material was administered intraperitoneally.
f. Number of times of administering the positive control material: The positive control material was administered once, the same as the test materials.

### 3. Preparation and administration of test materials

### a. Preparation of test materials

The peptide of SEQ ID NO: 11 (2 mg) was homogeneously dissolved in saline (20 mL) and stored in 2 mL aliquots (Final concentration: 0.1 mg/mL).

The peptide of SEQ ID NO: 12 (2 mg) was homogeneously dissolved in saline (20 mL) and stored in 2 mL aliquots (Final concentration: 0.1 mg/mL).

b. Determination of dose of the test materials: The dose was determined considering that the typical dose of intraperitoneal administration for mice is 200 µL.

c. Administration route of the test materials: The test materials were administered intraperitoneally because it is a general route which allows for easy systematic administration and absorption.

d. Method of administering the test materials: The test materials were administered intraperitoneally using a 1 mL syringe.

e. Number of times of administering the test materials: The test materials were administered once.

### 4. Configuration of test groups

The test groups were configured as described below.

**[Table 14]**

| Configuration | Administered material | Amount of administration | Administration concentration | Dose | route | n= |
|---|---|---|---|---|---|---|
| Negative control | Saline | - | - | | | 7 |
| Test group | SEQ ID NO: 11 | 1 mg/kg | 0.1 mg/mL | 200 µL | I.P | 7 |
| | SEQ ID NO: 12 | 1 mg/kg | 0.1 mg/mL | | | 7 |
| Positive control | Morphine | 10 mg/kg | 1 mg/mL | | | 7 |

### 5. Test method

a. The test animals were acclimated to the chamber of the measuring equipment three times for more than one hour each time according to the allodynia response behavior test, and after acclimation to the equipment was completed, the basic pain response value (baseline) was measured by the Von Frey test.

### b. CCI pain modeling

The test animals whose basic pain response value was measured were anesthetized, and the hair on the right sciatic nerve region of the test animals was removed to expose the skin. After disinfection, the skin and muscle layer were incised to find the sciatic nerve. The sciatic nerve was lightly tied two to three times using silk suture. The incised region was sutured again and disinfected.

### c. Confirmation of pain induction

On the postoperative Day 3 and Day 7, the Von Frey test was performed according to the allodynia response behavioral experiment to confirm the pain induction.

### d. Drug administration and confirmation of analgesic efficacy

After confirming that pain was induced, the test drug for each group was administered intraperitoneally.

Two hours after the administration of the test drug, the pain response was measured by performing the Von Frey test according to the allodynia response behavioral experiment.

The drug was administered as a single dose once a day, and the allodynia response was measured before and after the drug administration to confirm changes in the pain caused by the drug administration.

### e. Statistical analysis processing

Statistics were analyzed using T-test and expressed as Mean ± SEM (*=p<0.05, **=p<0.01, ***=p<0.001).

### 6. Test results

Currently, the number of chronic pain patients is gradually increasing due to various causes, and non-steroidal anti-inflammatory drugs (NSAID), GABA analogue drugs, narcotic analgesics or the like are being used as therapeutics for chronic pain. In particular, narcotic analgesics such as morphine are used for severe pain such as chronic pain and cancer pain.

The present experiment was performed to determine whether the administration of the peptide of SEQ ID NO: 11 or the peptide of SEQ ID NO: 12 exhibits a pain alleviating effect compared to the narcotic analgesic morphine.

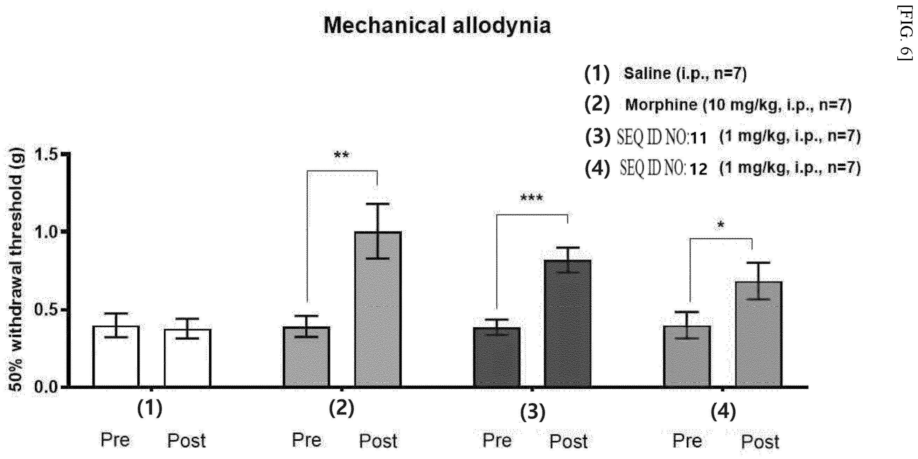

As a result of the experiment, the group administered with the peptide of SEQ ID NO: 11 or the peptide of SEQ ID NO: 12 showed a pain alleviating effect in which the pain behavioral response threshold increased after the administration compared to before the administration, and statistical significance was shown (see FIG. 6: Mean ± SEM, *= p<0.05, **=p<0.01, ***=p<0.001).

As a result of the above experiment, it could be seen that the administration of the peptide according to the present invention exhibits a similar effect at a low concentration compared to the narcotic analgesic morphine. This means that the peptide of the present invention can be effectively used as a pain alleviating medicine in replacement of narcotic analgesics.

### Example F. Confirmation of pain alleviating effect in SNL pain model

### 1. Test system information

**[Table 15]**

| | Description |
|---|---|
| Species | Rat |
| Line | S D rat |
| Sex | Male |
| Number of animals introduced | 2 5 |
| Supplier | YoungBIo |
| Age | Six weeks |
| Reason for selecting the test system | An inbreeding animal commonly used in non-clinical tests |

### 2. Preparation and administration of control materials

a. Preparation of negative control material: A complete saline solution product (0.9% NaCl) was used.
b. Method of administering the negative control material: The negative control material was administered intraperitoneally.
c. Number of times of administering the negative control material: The negative control material was administered once, the same as the test materials.
d. Preparation of positive control material: A complete morphine product (1 mg/mL) was used.
e. Method of administering the positive control material: The positive control material was administered intraperitoneally.
f. Number of times of administering the positive control material: The positive control material was administered once, the same as the test materials.

### 3. Preparation and administration of test materials

### a. Preparation of test materials

The peptide of SEQ ID NO: 11 (2 mg) was homogeneously dissolved in saline (10 mL) and used (Final concentration: 0.2 mg/mL).

The peptide of SEQ ID NO: 12 (2 mg) was homogeneously dissolved in saline (10 mL) and used (Final concentration: 0.2 mg/mL).

b. Determination of dose of the test materials: The dose was determined considering that the typical dose of intraperitoneal administration for rats is 2 mL.

c. Administration route of the test materials: The test materials were administered intraperitoneally because it is a general route which allows for easy systematic administration and absorption.

d. Method of administering the test materials: The test materials were administered intraperitoneally using a 3 mL syringe.

e. Number of times of administering the test materials: The test materials were administered once.

### 4. Configuration of test groups

The test groups were configured as described below.

**[Table 16]**

| Configuration | Administered material | Amount of administration | Administration concentration | Dose | route | n= |
|---|---|---|---|---|---|---|
| control | - | - | - | | | 5 |
| Negative control | Saline | - | - | | | 5 |
| Test group | SEQ ID NO: 11 | 2 mg/kg | 0.2 mg/mL | 2ml | I.P. | 5 |
| | SEQ ID NO: 12 | 2 mg/kg | 0.2 mg/mL | | | 5 |
| Positive control | Morphine | 10 mg/kg | 1 mg/mL | | | 5 |

### 5. Test method

a. The test animals were acclimated to the chamber of the measuring equipment three times for more than one hour each time according to the allodynia response behavior test, and after acclimation to the equipment was completed, the basic pain response value (baseline) was measured by the Von Frey test.

### b. SNL pain modeling

The test animals whose basic pain response value was measured were anesthetized, and the hair on the left spinal nerve region of the test animals was removed to expose the skin. After disinfection, the skin and muscle layer were incised to find the left 5th lumbar spinal nerve. The left 5th lumbar spinal nerve was lightly tied once using silk suture. The incised region was sutured again and disinfected.

### c. Confirmation of pain induction

On the postoperative Day 1, Day 7, and Day 14, the Von Frey test was performed according to the allodynia response behavioral experiment to confirm the pain induction.

### d. Drug administration and confirmation of analgesic efficacy

After confirming that pain was induced, the test drug for each group was administered intraperitoneally.

One hour, three hours, and seven hours after the administration of the test drug, the pain response was measured by performing the Von Frey test according to the allodynia response behavioral experiment.

The drug was administered as a single dose once a day, and the allodynia response was measured before and after the drug administration to confirm changes in the pain caused by the drug administration.

### e. Statistical analysis processing

Statistics were analyzed using T-test and expressed as Mean ± SEM (*=p<0.05, ***=p<0.001).

### 6. Test results

Chronic pain is caused by direct damage to nerves or inflammation, and is known to be very difficult to treat because it progresses through the simultaneous and complex action of several mechanisms. Neuropathic pain, which is a representative type of chronic pain, is one of the chronic pain syndromes that may be caused by various causes such as ischemic damage due to trauma or compression on the nerve, toxic metabolites, and inflammation, and is accompanied by symptoms such as spontaneous pain, hyperalgesia, and allodynia. Among neuropathic pain models, the spinal nerve ligation model is used in many drug administration experiments because it induces distinct and continuous mechanical allodynia and it effectively induces mechanical allodynia better than other nerve injury models.

The present experiment was performed to determine whether the administration of the peptide of SEQ ID NO: 11 or the peptide of SEQ ID NO: 12 exhibits a pain alleviating effect on the allodynia found in the SNL pain animal model compared to the narcotic analgesic morphine.

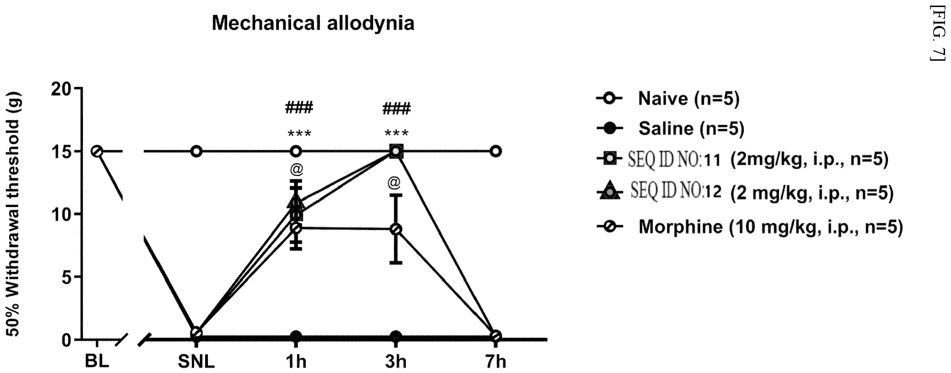

As a result of the experiment, the group administered with the peptide of SEQ ID NO: 11 or the peptide of SEQ ID NO: 12 showed a pain alleviating effect in which the pain behavioral response threshold increased after the administration compared to before the administration, and statistical significance was shown (see FIG. 7: Mean ± SEM, *= p<0.05, ***=p<0.001).

As a result of the above experiment, it could be seen that the administration of the peptide of SEQ ID NO: 11 or the peptide of SEQ ID NO: 12 appears to inhibit the allodynia found in the spinal nerve ligation model, and this means that the peptide of the present invention can be effectively used as a pain alleviating medicine in replacement of narcotic analgesics that are often used as therapeutics for neuropathic pain.

### Example G. Confirmation of pain alleviating effect in CIPN pain model

### 1. Test system information

**[Table 17]**

| | Description |
|---|---|
| Species | Rat |
| Line | S D rat |
| Sex | Male |
| Number of animals introduced | 17 |
| Supplier | YoungBIo |
| Age | Six weeks |
| Reason for selecting the test system | An inbreeding animal commonly used in non-clinical tests |

### 2. Preparation and administration of control materials

a. Preparation of negative control material: A complete saline solution product (0.9% NaCl) was used.
b. Method of administering the negative control material: The negative control material was administered intraperitoneally.
c. Number of times of administering the negative control material: The negative control material was administered once, the same as the test materials.
d. Preparation of positive control material: Prebabalin was homogeneously dissolved in a saline solution at a concentration of 30 mg/10 mL and used.
e. Method of administering the positive control material: The positive control material was administered intraperitoneally.
f. Number of times of administering the positive control material: The positive control material was administered once, the same as the test materials.

### 3. Preparation and administration of test materials

### a. Preparation of test materials

The peptide of SEQ ID NO: 11 (4 mg) was homogeneously dissolved in saline (10 mL) and used (Final concentration: 0.4 mg/mL).

The peptide of SEQ ID NO: 11 (2 mg) was homogeneously dissolved in saline (10 mL) and used (Final concentration: 0.2 mg/mL).

b. Determination of dose of the test materials: The dose was determined considering that the typical dose of intraperitoneal administration for rats is 2 mL.

c. Administration route of the test materials: The test materials were administered intraperitoneally because it is a general route which allows for easy systematic administration and absorption.

d. Method of administering the test materials: The test materials were administered intraperitoneally using a 3 mL syringe.

e. Number of times of administering the test materials: The test materials were administered once.

### 4. Configuration of test groups

The test groups were configured as described below.

**[Table 18]**

| Configuration | Administered material | Amount of administration | Administration concentration | Dose | route | n= |
|---|---|---|---|---|---|---|
| control | - | - | - | | | 2 |
| Negative control | Saline | - | - | | | 3 |
| Test group | SEQ ID NO: 11 | 4 mg/kg | 0.4 mg/mL | 2ml | I.P | 4 |
| | SEQ ID NO: 12 | 2 mg/kg | 0.2 mg/mL | | | 4 |
| Positive control | Pregabalin | 30 mg/kg | 3 mg/mL | | | 4 |

### 5. Test method

a. The test animals were acclimated to the chamber of the measuring equipment three times for more than one hour each time according to the allodynia response behavior test, and after acclimation to the equipment was completed, the basic pain response value (baseline) was measured by the Von Frey test.

### b. Chemotherapy induced peripheral neuropathy (CIPN) pain modeling

Paclitaxel was dissolved at 2 mg/10 ml in a solution prepared by mixing cremophor EL and absolute ethanol at a 1:1 ratio, and the resulting solution was administered intraperitoneally in the amount of 2 ml each. Chemotherapy-induced pain was induced by administering paclitaxel once a day at 24-hour intervals for 4 days.

### c. Confirmation of pain induction

Day 1, Day 7, and Day 14 after the pain induction, the Von Frey test was performed according to the allodynia response behavioral experiment to confirm the pain induction.

### d. Drug administration and confirmation of analgesic efficacy

After confirming that pain was induced, the test drug for each group was administered intraperitoneally.

One hour, three hours, and seven hours after the administration of the test drug, the pain response was measured by performing the Von Frey test according to the allodynia response behavioral experiment.

The drug was administered as a single dose once a day, and the allodynia response was measured before and after the drug administration to confirm changes in the pain caused by the drug administration.

### e. Statistical analysis processing

Statistics were analyzed using T-test and expressed as Mean ± SEM (*=p<0.05, ***=p<0.001).

### 6. Test results

With the increase of average life expectancy, the incidence of cancer increases, and cancer treatment advances, and the number of cancer survivors is increasing. In this situation, improving the quality of life of cancer patients can be said to be a very important topic. Chemotherapy induced peripheral neuropathy (CIPN) is differentiated from cancer pain which is caused when cancer directly invades nerve tissues and compresses the nerve or metastasizes to the bone, and it is found in about 40% of patients undergoing complex chemotherapy.

The present experiment was performed to determine whether the administration of the peptide of SEQ ID NO: 11 or the peptide of SEQ ID NO: 12 exhibits a pain alleviating effect on the allodynia found in the CIPN pain animal model compared to pregabalin.

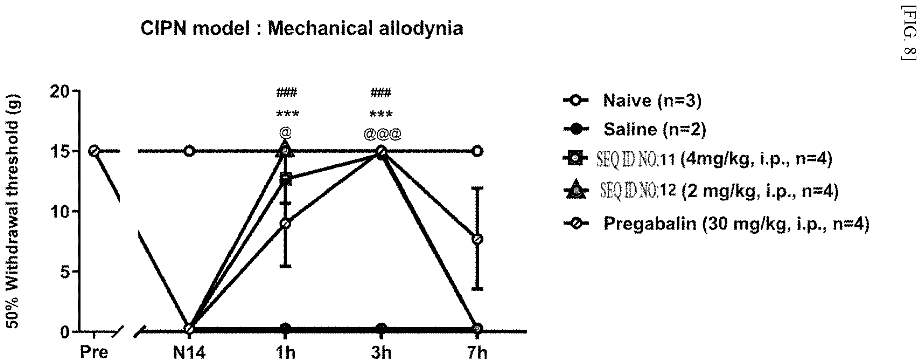

As a result of the experiment, the group administered with the peptide of SEQ ID NO: 11 or the peptide of SEQ ID NO: 12 showed a pain alleviating effect in which the pain behavioral response threshold increased after the administration compared to before the administration, and statistical significance was shown (see FIG. 8: Mean ± SEM, *= p<0.05, ***=p<0.001).

From the above results, it appears that the administration of the peptide of SEQ ID NO: 11 or the peptide of SEQ ID NO: 12 appears to inhibit the allodynia found in the chemotherapy induced peripheral neuropathy model, and this means that the peptide of the present invention can be effectively used as a pain alleviating medicine in replacement of analgesics such as gabapentin and pregabalin that are clinically used for the treatment of CIPN.

### INDUSTRIAL APPLICABILITY

The present invention can be effectively used as a medicine for alleviating pain.

### [Sequence list free text]

SEQ ID NO:1 : MLYFNDKQQI IY
SEQ ID NO: 2 : PINMLYFNDKQQIIYGKID
SEQ ID NO:3 : GINMLYFNDKQQIIYGKIN
SEQ ID NO: 4 : YCSGQCYMFM QKY
SEQ ID NO: 5 : YKANYCSGQC Y
SEQ ID NO: 6 : SGQCYMFMQK Y
SEQ ID NO: 7 : YKANYCSGQC EY
SEQ ID NO: 8 : SGQCEYMFMQ KY
SEQ ID NO: 9 : AGQCYMFMQK Y
SEQ ID NO: 1 0 : SGQAYMFMQK Y
SEQ ID NO: 1 1 : YGQCYMWMQK W
SEQ ID NO: 1 2 : YGQCYMYMQK W
SEQ ID NO: 1 3 : YGQCYMWMQY W
SEQ ID NO: 1 4 : YGQCYMWMQW W
SEQ ID NO: 1 5 : YGQCYMWMQG W
SEQ ID NO: 1 6 : YGQCYMWMQK Y
SEQ ID NO: 1 7 : YGQCYMWMQE W
SEQ ID NO: 1 8 : YGMCYMWMQK W
SEQ ID NO: 1 9 : YGRCYMWMQK W
SEQ ID NO: 2 0 : YGQCYMWFQK W
SEQ ID NO: 2 1 : ICYMWMQK W
SEQ ID NO: 2 2 : YGQCYMWMQH W
SEQ ID NO: 23 : Y G Q C Y M W Y Q K W
SEQ ID NO: 24 : YGQCYMWSQK W
SEQ ID NO: 25 : YGGCYMWMQK W
SEQ ID NO: 26 : YKQCYMWMQK W
SEQ ID NO: 27 : YGTCYMWMQK W
SEQ ID NO: 28 : YGQCYMWGQK W
SEQ ID NO: 29 : YGQCYMWMQS W
SEQ ID NO: 30 : YGQCYMWMQC W
SEQ ID NO: 31 : YGQCYMWMQ A W
SEQ ID NO: 32 : YGQWYMWMQK W
SEQ ID NO: 33 : YGQMYMWMQK W
SEQ ID NO: 34 : YGLCYMWMβK W
SEQ ID NO: 35 : YGQCYMWHQK W
SEQ ID NO: 36 : YSQCYMWMQK W
SEQ ID NO: 37 : YGQCYMWMQM W
SEQ ID NO: 38 : YGQPYMWMQK W
SEQ ID NO: 39 : YGQCYMWPQK W
SEQ ID NO: 40 : YGQGYMWMQK W
SEQ ID NO: 41 : YGSCYMWMQK W
SEQ ID NO: 42 : YLQCYMWMQK W
SEQ ID NO: 43 : YKAAYCSGQC Y
SEQ ID NO: 44 : SGACYMFMQK Y
SEQ ID NO: 45 : SGQCYAFMQK Y
SEQ ID NO: 46 : SGQCYMFMQA Y
SEQ ID NO: 47 : SGQCYMWMQK Y
SEQ ID NO: 48 : SGQCYMWMQK W
SEQ ID NO: 49 : SGQCYMYMQK Y
SEQ ID NO:50:NLGLDCDEHS SESRCCRYPL TVDFEAFGWD WIIAPKRYKA NYCSGQCEYM FMQKYPHTHL VQQANPRGSA GPCCTPTKMS PINMLYFNDK QQIIYGKIPG MVVDRCGCS
SEQ ID NO: 51 : LYFNDKQQTI Y
SEQ ID NO: 52 : NMLYFNDKQQ IIYGK
SEQ ID NO: 53 : PINMLYFNDK QQIIYGKIP

## Claims

1. A peptide comprising any one sequence of SEQ ID NOS: 1 to 42.

2. A peptide comprising any one sequence represented by Formula 1:
[Formula 1] X1-X2-X3-X4-Y-M-X7-X8-Q-X10-W
wherein
X1 is A, S or Y,
X2 is G, K, S or L,
X3 is Q, M, R, I, T, L or S,
X4 is A, C, W, M, P or G,
X7 is F, W or Y,
X8 is M, F, Y, S, G, H or P, and
X10 is any amino acid.

3. The peptide according to claim 2, wherein
X1 is Y,
X2 is G,
X3 is Q,
X4 is C,
X7 is W or Y, and
X8 is a hydrophobic amino acid.

4. The peptide according to claim 3, wherein the hydrophobic amino acid is M, F or Y.

5. The peptide according to claim 2, wherein the X10 is K, Y, W, G, E, H, S, C, A or M.

6. An oligopeptide comprising the peptide according to claim 1.

7. An oligopeptide comprising the peptide according to any one of claims 2 to 5.

8. A pharmaceutical composition for treating TRPV1-mediated diseases, comprising the peptide according to any one of claims 1 to 5.

9. A pharmaceutical composition for treating pain, comprising the peptide according to claim 2.

10. A pharmaceutical composition for treating pain, comprising the peptide according to any one of claims 2 to 5.

11. A polynucleotide encoding the peptide according to claim 1.

12. A polynucleotide encoding the peptide according to any one of claims 2 to 5.

13. The peptide according to any one of claims 1 to 5, wherein an N-terminus and/or a C-terminus are capped to protect from protease enzymes.

14. The peptide according to claim 13, wherein the N-terminus is capped with lipoic acid.

15. The peptide according to claim 13, wherein the C-terminus is capped with p-nitroanilide.
